# EUROPEAN PATENT SPECIFICATION

(11) **EP 3 188 707 B1**
(45) Date of publication and mention of the grant of the patent: **11.05.2022**
(21) Application number: 14766317.3
(22) Date of filing: 01.09.2014
(51) Int. Cl.: A61K 8/27, A61Q 11/00, A61K 8/97, A61K 8/31, A61K 8/33, A61K 8/34, A61K 8/35, A61K 8/37, A61K 8/9789

(54) **ZINC-CONTAINING COMPOSITIONS WITH ESSENTIAL OILS**
ZINK-ENTHALTENDE VERBINDUNGEN MIT ESSENTIELLEN OELEN
COMPOSITIONS CONTENANT DU ZINC AVEC DES HUILES ESSENTIELLES

(43) Date of publication of application: 12.07.2017
(73) Proprietor: Colgate-Palmolive Company, New York, NY 10022 (US)
(72) Inventor: PRENCIPE, Michael, Princeton Junction, New Jersey 08550 (US); FISHER, Steven, Middlesex, New Jersey 08846 (US); TAMBS, Gary, Piscataway NJ 08854 (US)
(74) Representative: Wibbelmann, Jobst
(86) International application number: PCT/US2014/053629
(87) International publication number: WO 2016/036341

(56) References cited:
- WO-A1-97/40812
- WO-A1-2006/071755
- WO-A1-2011/068815
- WO-A1-2012/076310
- WO-A1-2013/066403
- WO-A2-00/00166
- WO-A2-2011/019342
- US-A1- 2008 253 976
- US-A1- 2012 014 883
- Anonymous: "GNPD - Maximum Bio-Active Super Fresh Toothpaste", , 1 March 2014 (2014-03-01), XP055186450, Retrieved from the Internet: URL:http://www.gnpd.com/sinatra/recordpage /2337697/from_search/lQ5ZuZM2pG/ [retrieved on 2015-04-28]
- Anonymous: "GNPD - Chinese Herbal Odour Removing Toothpaste", , 1 June 2014 (2014-06-01), XP055186451, Retrieved from the Internet: URL:http://www.gnpd.com/sinatra/recordpage /2465795/from_search/lQ5ZuZM2pG/ [retrieved on 2015-04-28]

## Description

### BACKGROUND

Dental plaque is a biofilm that adheres to tooth and other oral surfaces, particularly at the gingival margin, and is implicated in the occurrence of gingivitis, periodontitis, caries and other forms of periodontal disease. Various antibacterial agents can retard the growth of bacteria and thus reduce the formation of biofilm on oral surfaces. Zinc and other metal compounds/salts have been previously used as antibacterial agents. Without being bound by any theory, free zinc ions are believed to provide antibacterial efficacy by inhibition of glucose metabolism and/or interaction with the bacterial cell wall, reducing bacterial colonization of the oral cavity (as discussed in Cummins D., J Clin Periodontal 1991; 18; 455-461). An insoluble zinc compound, zinc oxide, could also deliver strong antibacterial efficacy during tooth brushing.

WO 2012/076310 A1 discloses an oral care composition comprising (i) 0.05 to 10% of an antimicrobial essential oil comprising thymol and terpineol, such that the composition comprises 0.01 to 5 % by weight thymol, and 0.01 to 5 % by weight terpineol; (ii) 0.01 to 10% of a zinc salt; and (iii) an orally acceptable base.

WO9740812A1 discloses an improved oral composition comprising a bacteriostatic and anticalculus effective amount of zinc ion provided by one or more zinc-containing compounds and an antimicrobial effective amount of one or more essential oils.

WO 00/00166 A2 discloses An oral composition comprising: thymol, zinc ion in an amount from about 0.005 to about 0.095% by weight of the composition, a sweetener; and an aqueous vehicle.

US 2008/253976 A1 discloses a blend of essential oil components comprising a first acyclic component selected from citral, neral, geranial, geraniol and nerol and a second cyclic-containing component selected from eucalyptol, eugenol and carvacrol and used in personal care compositions at a level of at least about 0.02% by weight to provide effective antimicrobial activity.

WO 2011/068815 A1 discloses an oral composition comprising: a combination of extracts comprising an extract from Zizyphus joazeiro and a natural extract other than the extract from Zizyphus joazeiro; and an orally acceptable carrier.

WO 2006/071755 A1 discloses an oral care composition according to claim 1, wherein the active ingredient consists essentially of one or more active compounds from an extract of oregano.

US 2012/014883 A1 discloses a composition comprising one or more derivatives of parent essential oil compounds selected from aldehydes, ketones, alcohols, phenolics or acids, for use in personal care compositions at a level of at least about 0.02% by weight to provide effective antimicrobial activity

The document "GNPD - Maximum Bio-Active Super Fresh Toothpaste", URL http://www.gnpd.com/sinatra/recordpage/2337697/from_search/lQ5ZuZM2pG/, discloses antibacterial oral care compositions with zinc citrate, thymol, limonene.

The document "GNPD - Chinese Herbal Odour Removing Toothpaste", URL: http://www.gnpd.com/sinatra/recordpage/2465795/from_search/lQ5ZuZM2pG/, discloses antibacterial oral care compositions with zinc citrate, thymol, borneol.

WO 2011/019342 A2 discloses an oral care composition comprising a sesquiterpenoid and an antimicrobial agent, wherein the sesquiterpenoid and the antimicrobial agent are present in an amount effective to inhibit and/or degrade a biofilm in the oral cavity.

WO 2013/066403 A1 discloses an antimicrobial composition comprising: (a) from about 0.5% (w/w) to about 50% (w/w) benzyl alcohol; (b) from about 0.01 % (w/w) to about 5% (w/w) essential oil; and (c) from about 0.5% (w/w) to about 10% (w/w) botanical extract selected from the group consisting of wasabi. extract, honeysuckle extract, cedar wood extract, aspen bark extract, willow bark extract, tobacco extract, and combinations thereof

It would be desirable to provide an oral care composition which exhibits even greater biofilm reduction efficacy than previously-known compositions.

### BRIEF SUMMARY

In a first aspect, the present invention provides an oral care composition comprising: (a) a zinc ion source, wherein the composition comprises from 0.1 to 2.0 weight % zinc oxide and from 0.20 to 1.0 weight % zinc citrate; and (b) at least two active ingredients, wherein said active ingredients are selected from the following: bisabolol; citral; carvacrol; thymol; and an extract of oregano, wherein the active compound from said extract of oregano is selected from carvacrol, thymol, limonene, pinene, p-cymene, caryophyllene, and mixtures thereof; and an extract of rosemary, wherein the active compound from said extract of rosemary is selected from α-pinene, β-pinene, borneol, bornyl acetate, camphor, camphene, 1,8-cineole, limonene, and mixtures thereof.

Optionally, the total concentration of the zinc ion source is from 0.5 to 2.0 weight %, based on the total weight of the composition.

Optionally, the active ingredients are carvacrol and thymol. Optionally, the composition comprises carvacrol. Optionally, the composition comprises thymol.

Optionally, the composition comprises citral.

Optionally, the composition comprises bisabolol.

Optionally, the composition comprises bisabolol and citral. Further optionally, the ratio of citral to bisabolol is from 1:1 to 3:1 by weight; optionally 2:1 by weight.

Optionally, the composition comprises bisabolol and carvacrol. Further optionally, the ratio of bisabolol to carvacrol is from 1:2 to 2:1 by weight; optionally 1.5:1 by weight.

Optionally, the composition comprises bisabolol and thymol.

Optionally, the composition comprises citral and carvacrol.

Optionally, the composition comprises citral and thymol.

Optionally, the composition comprises carvacrol and thymol. Further optionally, the ratio of thymol to carvacrol is from 8:1 to 13:1 by weight; optionally 11.25:1 by weight.

The active compounds from an extract of oregano are carvacrol, thymol, limonene, pinene, p-cymene and caryophyllene. Further optionally, the oral care composition comprises an extract of oregano, the extract of oregano comprising carvacrol, thymol, limonene, pinene, p-cymene and caryophyllene.

Optionally, the oral care composition comprises an extract of oregano, the extract of oregano comprising 50 to 70 weight % carvacrol, 10 to 35 weight % thymol and 10 to 20 weight % p-cymene, based on the weight of the extract of oregano.

Optionally, the composition comprises bisabolol and the extract of oregano. Further optionally, the ratio of the extract of oregano to bisabolol is from 2:1 to 6:1 by weight; optionally 4.4:1 by weight.

Optionally, the composition comprises citral and the extract of oregano.

The active compounds from an extract of rosemary are α-pinene, β-pinene, borneol, bornyl acetate, camphor, camphene, 1,8-cineole and limonene. Further optionally, the oral care composition comprises an extract of rosemary, the extract of rosemary comprising α-pinene, β-pinene, borneol, bornyl acetate, camphor, camphene, 1,8-cineole and limonene.

Optionally, the oral care composition comprises an extract of rosemary, the extract of rosemary comprising 25 to 40 weight % α-pinene, 5 to 20 weight % β-pinene, and 10 to 25 weight % camphor, based on the weight of the extract of rosemary.

Optionally, the composition comprises bisabolol and the extract of rosemary. Further optionally, the ratio of the extract of rosemary to bisabolol is from 40:1 to 60:1 by weight; optionally 48:1 by weight.

Optionally, the composition comprises citral and the extract of rosemary.

Optionally, the composition comprises carvacrol and the extract of rosemary. Further optionally, the ratio of the extract of rosemary to carvacrol is from 70:1 to 80:1 by weight; optionally 75:1 by weight.

Optionally, the composition comprises thymol and the extract of rosemary.

Optionally, the composition comprises the extract of rosemary and an extract of oregano. Further optionally, the extract of oregano comprises carvacrol, thymol, limonene, pinene, p-cymene and caryophyllene.

Optionally, the extract of oregano comprises 50 to 70 weight % carvacrol, 10 to 35 weight % thymol and 10 to 20 weight % p-cymene, based on the weight of the extract of oregano.

Optionally, in addition to the zinc oxide and the zinc citrate, the zinc ion source may comprise a zinc compound selected from zinc acetate, zinc gluconate, zinc glycinate, zinc sulfate, zinc phosphate and sodium zinc citrate. Still further optionally, the molar ratio of zinc oxide to zinc citrate can be up to 3:1. Yet further optionally, the molar ratio of zinc oxide to zinc citrate is from 1:1 to 3:1, optionally 2:1.

The composition comprises from 0.1 to 2.0 weight % zinc oxide and from 0.20 to 1.0 weight % zinc citrate; optionally from 0.5 to 1.5 weight % zinc oxide and from 0.25 to 0.75 weight % zinc citrate.

Optionally, the composition is a dentifrice, a toothpaste, a gel, a tooth powder, a mouthwash, a mouthrinse, a lozenge, a tablet, a spray, a gum, or a film.

In a second aspect, the present invention provides an oral care composition of the present invention, for use in reducing or inhibiting biofilm formation in an oral cavity.

In a third aspect, the present invention provides a method of reducing or inhibiting biofilm formation in an oral cavity, the method comprising contacting the oral cavity with an oral care composition of the present invention.

In a fourth aspect, the present invention provides the use, in an oral care composition of the present invention, of a combination of (a) a zinc ion source; and (b) at least two active ingredients to reduce or inhibit biofilm formation in an oral cavity..

### DETAILED DESCRIPTION

As used throughout, ranges are used as shorthand for describing each and every value that is within the range. Any value within the range can be selected as the terminus of the range.

Unless otherwise specified, all percentages and amounts expressed herein and elsewhere in the specification should be understood to refer to percentages by weight. The amounts given are based on the active weight of the material. As referred to herein, "ppm" (parts per million) refers to ppm by weight unless otherwise indicated. In addition, all ratios expressed herein refer to ratios by weight unless otherwise indicated.

Unless otherwise specified, all experiments described herein are conducted at 25°C and under atmospheric pressure.

As discussed above, it would be desirable to provide an oral care composition which exhibits greater biofilm reduction efficacy than previous oral care compositions.

Therefore, in one aspect of the present invention, there is provided an oral care composition comprising: (a) a zinc ion source, wherein the composition comprises from 0.1 to 2.0 weight % zinc oxide and from 0.20 to 1.0 weight % zinc citrate; and (b) at least two active ingredients, wherein said active ingredients are selected from the following: bisabolol; citral; carvacrol; thymol; and an extract of oregano, wherein the active compound from said extract of oregano is selected from carvacrol, thymol, limonene, pinene, p-cymene, caryophyllene, and mixtures thereof; and an extract of rosemary, wherein the active compound from said extract of rosemary is selected from α-pinene, β-pinene, borneol, bornyl acetate, camphor, camphene, 1,8-cineole, limonene, and mixtures thereof.

In some embodiments, the total concentration of the at least two active ingredients in the oral care composition is from 50 to 12,000 ppm (0.005 to 1.2 weight %), from 100 to 12,000 ppm (0.01 to 1.2 weight %), from 500 to 10,000 ppm (0.05 to 1 weight %), from 700 to 6000 ppm (0.07 to 0.6 weight %), from 900 to 5500 ppm (0.09 to 0.55 weight %), from 1000 to 5000 ppm (0.1 to 0.5 weight %), from 3000 to 5000 ppm (0.3 to 0.5 weight %); or 1000 ppm (0.1 weight %), 3000 ppm (0.3 weight %), or 5000 ppm (0.5 weight %). In some embodiments, the total concentration of the at least two active ingredients in the oral care composition is from 2500 to 5500 ppm (0.25 to 0.55 weight %), from 4500 to 5500 ppm (0.45 to 0.55 weight %), from 4800 to 5200 ppm (0.48 to 0.52 weight %), or 5000 ppm (0.5 weight %).

Terpenes are hydrocarbons formally derived from the combination of one or more five-carbon isoprene units, CH₂=C(CH₃)CH=CH₂ (2-methylbuta-1,3-diene, molecular formula C₅H₈). The isoprene units may be linked together to form linear chains, or may be arranged to form rings (for example, benzene rings). Terpenoids (sometimes referred to as "isoprenoids") can be thought of as being modified terpenes, e.g. containing additional functional groups (such as, for example, alcohol or aldehyde groups) or wherein methyl groups have been moved or removed. As for terpenes, terpenoids can be classified according to the number of isoprene units from which they are formally derived:
- Hemiterpenoids: 1 isoprene unit (i.e. C₅ skeleton)
- Monoterpenoids: 2 isoprene units (i.e. C₁₀ skeleton)
- Sesquiterpenoids: 3 isoprene units (i.e. C₁₅ skeleton)
- Diterpenoids: 4 isoprene units (i.e. C₂₀ skeleton)
- Sesterterpenoids: 5 isoprene units (i.e. C₂₅ skeleton)
- Triterpenoids: 6 isoprene units (i.e. C₃₀ skeleton)
- Tetraterpenoids: 8 isoprene units (i.e. C₄₀ skeleton)

A monoterpenoid aldehyde which may be used in the compositions of the present invention is citral (also known as 3,7-dimethyl-2,6-octadienal or lemonal). There are two double-bond stereoisomers of citral: the *E*-isomer (which is known as geranial or citral A); and the Z-isomer (which is known as neral or citral B). In the present invention, the citral is a mixture of these two isomers at a ratio of 60:40 *E*-isomer to Z-isomer. The structures of these two isomers is shown below:

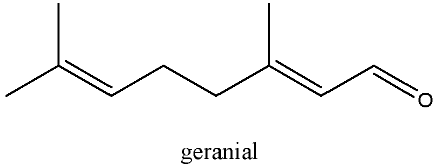

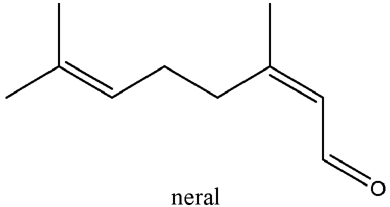

In some embodiments, the oral care composition comprises citral. In some embodiments, citral is present in the oral care composition in an amount of from 500 to 4000 ppm, 600 to 3600 ppm, 1500 to 3500 ppm, 2000 to 3500 ppm, or 3200 to 3400 ppm.

Monoterpenoid phenols which may be used in the compositions of the present invention include carvacrol and thymol. Thymol (also known as 2-isopropyl-5-methylphenol) is isomeric with carvacrol (also known as 5-isopropyl-2-methylphenol, or cymophenol). The structures of these two compounds are shown below:

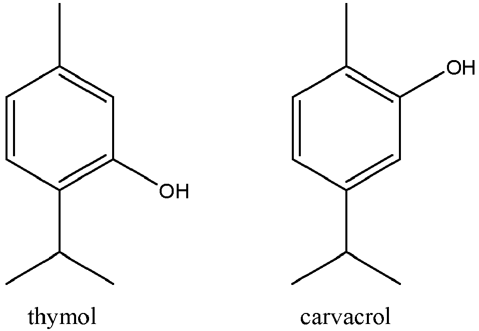

In some embodiments, the oral care composition comprises carvacrol. In other embodiments, the oral care composition comprises thymol.

In some embodiments, carvacrol is present in the oral care composition in an amount of from 5 to 3000 ppm, from 10 to 2500 ppm, or from 10 to 2000 ppm. In some embodiments, carvacrol is present in the oral care composition in an amount of from 80 to 2000 ppm. In other embodiments, carvacrol is present in the oral care composition in an amount of from 10 to 75 ppm, from 35 to 70 ppm, or from 50 to 70 ppm.

In some embodiments, thymol is present in the oral care composition in an amount of from 500 to 5000 ppm, from 800 to 4800ppm, from 900 to 4600 ppm, from 2600 to 4600 ppm, or from 4500 to 4600 ppm.

In certain embodiments, the oral care composition comprises thymol and carvacrol. In some embodiments, the ratio of thymol to carvacrol is from 5:1 to 15:1 by weight, from 6:1 to 14:1 by weight, from 8:1 to 13:1 by weight, from 9:1 to 12.5:1 by weight, from 10:1 to 12:1 by weight, or 11.25:1 by weight. In some embodiments, the oral care composition comprises from 50 to 450 ppm carvacrol and from 800 to 4800 ppm thymol; from 80 to 410 ppm carvacrol and from 900 to 4600 ppm thymol; from 200 to 410 ppm carvacrol and from 2600 to 4600 ppm thymol; or from 390 to 410 ppm carvacrol and from 4500 to 4600 ppm thymol.

A sesquiterpenoid alcohol which may be used in the compositions of the present invention is bisabolol (also known as levomenol). There are two structural isomers of bisabolol: α-bisabolol and β-bisabolol. The structures of these isomers are shown below:

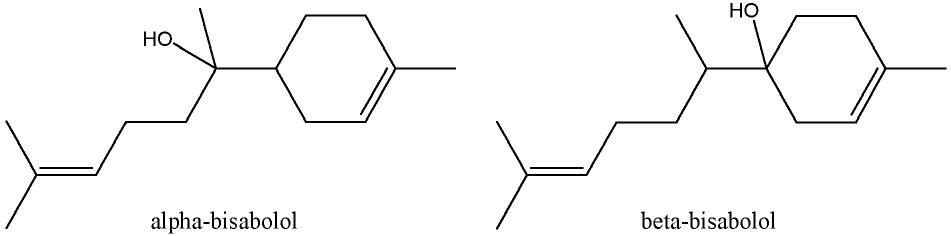

In certain embodiments of the present invention, the isomer of bisabolol which is present in the oral care compositions is α-bisabolol. There are two enantiomers of α-bisabolol: α-(-)-bisabolol and α-(+)-bisabolol. Of these enantiomers, α-(-)-bisabolol (illustrated below) is naturally occurring, and is found in German chamomile (*Matricaria recutita*) and *Myoporum crassifolium.* α-(+)-Bisabolol is also found in nature, but is rare. 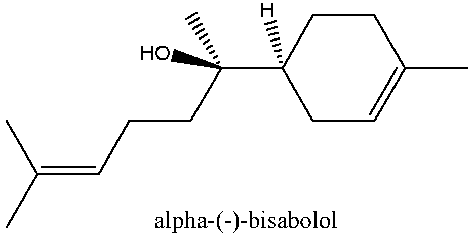 Synthetic bisabolol is usually a racemic mixture of the two enantiomers i.e. α-(±)-bisabolol. In certain embodiments of the present invention, the bisabolol which is present in the oral care compositions is racemic α-(±)-bisabolol.

In some embodiments, the oral care composition comprises bisabolol. In some embodiments, bisabolol is present in the oral care composition in an amount of from 10 to 3500 ppm, from 15 to 3100 ppm, or from 20 to 3050 ppm. In certain embodiments, bisabolol is present in the oral care composition in an amount of from 10 to 1000 ppm, from 20 to 950 ppm, or from 100 to 950 ppm. In other embodiments, bisabolol is present in the oral care composition in an amount of from 300 to 3500 ppm, from 1000 to 3200 ppm, or from 1500 to 3100 ppm.

In some embodiments, the oral care composition comprises bisabolol and citral. In certain embodiments, the ratio of citral to bisabolol is from 0.5:1 to 4:1 by weight, from 1:1 to 3:1 by weight, from 1.5:1 to 2.5:1 by weight, from 1.75:1 to 2.25:1 by weight, or 2:1 by weight. In some embodiments, the oral care composition comprises from 300 to 2000 ppm bisabolol and from 500 to 3500 ppm citral; from 300 to 1700 ppm bisabolol and from 600 to 3400 ppm citral; from 1000 to 1700 ppm bisabolol and from 1800 to 3400 ppm citral; or from 1500 to 1700 ppm bisabolol and from 3100 to 3400 ppm citral.

In some embodiments, the oral care composition comprises bisabolol and carvacrol. In certain embodiments, the ratio of bisabolol to carvacrol is from 1:3 to 3:1 by weight, from 0.5:1 to 2:1 by weight, from 1:1 to 2:1 by weight, from 1.25:1 to 1.75:1 by weight, or 1.5:1 by weight. In some embodiments, the oral care composition comprises from 500 to 3500 ppm bisabolol and from 250 to 2500 ppm carvacrol; from 600 to 3100 ppm bisabolol and from 300 to 2000 ppm carvacrol; from 1500 to 3100 ppm bisabolol and from 1000 to 2000 ppm carvacrol; or from 3000 to 3100 ppm bisabolol and from 1900 to 2000 ppm carvacrol.

In some embodiments, the oral care composition comprises bisabolol and thymol.

In some embodiments, the composition comprises citral and carvacrol.

In some embodiments, the composition comprises citral and thymol.

In some embodiments, the active compounds from an extract of oregano are cyclic monoterpenes, bicyclic monoterpenes, monoterpenoid phenols, bicylic sesquiterpenoids, and alkylbenzene compounds. In some embodiments, the active compounds from an extract of oregano are carvacrol, thymol, limonene, pinene, p-cymene and caryophyllene. Limonene is a cyclic monoterpene, pinene is a bicyclic monoterpene, caryophyllene is a bicylic sesquiterpenoid, and p-cymene is an alkylbenzene compound related to cyclic monoterpenes. In some embodiments, the oral care composition comprises an extract of oregano, the extract of oregano comprising carvacrol, thymol, limonene, pinene, p-cymene and caryophyllene. In certain embodiments, the extract of oregano comprises 50 to 70 weight % carvacrol, 10 to 35 weight % thymol and 10 to 20 weight % p-cymene, based on the weight of the extract of oregano; or 55 to 65 weight % carvacrol, 10 to 35 weight % thymol and 12 to 18 weight % p-cymene, based on the weight of the extract of oregano. In some embodiments, the extract of oregano comprises 61 weight % carvacrol and 15 weight % p-cymene, based on the weight of the extract of oregano.

In some embodiments, the oral care composition comprises bisabolol and the extract of oregano. In some embodiments, the ratio of the extract of oregano to bisabolol is from 1:1 to 7:1 by weight, from 2:1 to 6:1 by weight, from 3:1 to 5:1 by weight, from 4:1 to 4.5:1 by weight, or 4.4:1 by weight. In some embodiments, the oral care composition comprises from 150 to 1000 ppm bisabolol and from 500 to 4500 ppm extract of oregano; from 180 to 950 ppm bisabolol and from 800 to 4100 ppm extract of oregano; from 500 to 950 ppm bisabolol and from 2200 to 4200 ppm extract of oregano; or from 850 to 950 ppm bisabolol and from 3900 to 4100 ppm extract of oregano.

In some embodiments, the composition comprises citral and the extract of oregano.

The active compounds from an extract of rosemary are α-pinene, β-pinene, borneol, bornyl acetate, camphor, camphene, 1,8-cineole and limonene. Bomeol is a monoterpenoid alcohol, camphor is a monoterpenoid ketone, camphene is a bicyclic monoterpene, and 1,8-cineole (also known as eucalyptol) is a cyclic monoterpenoid ether. In some embodiments, the oral care composition comprises an extract of rosemary, the extract of rosemary comprising α-pinene, β-pinene, borneol, bornyl acetate, camphor, camphene, 1,8-cineole and limonene. In certain embodiments, the extract of rosemary comprises 25 to 40 weight % α-pinene, 5 to 20 weight % β-pinene, and 10 to 25 weight % camphor, based on the weight of the extract of rosemary; or 30 to 40 weight % α-pinene, 10 to 15 weight % β-pinene, and 10 to 15 weight % camphor, based on the weight of the extract of rosemary. In certain embodiments, the extract of rosemary comprises 36 weight % α-pinene, 13 weight % β-pinene, and 16 weight % camphor, based on the weight of the extract of rosemary.

In some embodiments, the oral care composition comprises bisabolol and the extract of rosemary. In some embodiments, the ratio of the extract of rosemary to bisabolol is from 40:1 to 60:1 by weight, from 45:1 to 55:1 by weight, from 46:1 to 50:1 by weight, or 48:1 by weight. In some embodiments, the oral care composition comprises from 10 to 150 ppm bisabolol and from 800 to 5200 ppm extract of rosemary; from 20 to 110 ppm bisabolol and from 970 to 5000 ppm extract of rosemary; from 50 to 110 ppm bisabolol and from 2800 to 5000 ppm extract of rosemary; or from 90 to 110 ppm bisabolol and from 4800 to 5000 ppm extract of rosemary.

In some embodiments, the composition comprises citral and the extract of rosemary.

In some embodiments, the composition comprises carvacrol and the extract of rosemary. In some embodiments, the ratio of the extract of rosemary to carvacrol is from 60:1 to 90:1 by weight, from 65:1 to 85:1 by weight, from 70:1 to 80:1 by weight, or 75:1 by weight. In some embodiments, oral care composition comprises from 5 to 80 ppm carvacrol and from 800 to 5300 ppm extract of rosemary; from 10 to 70 ppm carvacrol and from 980 to 5000 ppm extract of rosemary; from 30 to 70 ppm carvacrol and from 2800 to 5000 ppm extract of rosemary; or from 50 to 70 ppm carvacrol and from 4800 to 5000 ppm extract of rosemary.

In some embodiments, the composition comprises thymol and the extract of rosemary.

In some embodiments, the composition comprises the extract of rosemary and an extract of oregano. The extract of oregano can comprise carvacrol, thymol, limonene, pinene, p-cymene and caryophyllene. In certain embodiments, the extract of oregano comprises 50 to 70 weight % carvacrol, 10 to 35 weight % thymol and 10 to 20 weight % p-cymene, based on the weight of the extract of oregano.

In certain embodiments, the oral care composition comprises a combination of:
(a) bisabolol and citral,
(b) bisabolol and carvacrol,
(c) carvacrol and thymol,
(d) bisabolol and an extract of oregano,
(e) bisabolol and an extract of rosemary, or
(f) carvacrol and an extract of rosemary;
wherein the ratios and amounts of the bisabolol, citral, carvacrol, thymol, extract of oregano and extract of rosemary in each of combinations (a) to (f) may be as described in any of the above embodiments relating to these combinations.

In some embodiments, the total concentration of the at least one zinc ion source in the oral care composition is from 0.1 to 4.0 weight %, from 0.3 to 3.0 weight %, from 0.5 to 2.0 weight %, from 1.0 to 1.9 weight %, from 1.3 to 1.7 weight %, or 1.5 weight %, based on the total weight of the composition.

In certain embodiments, the zinc ion source comprises zinc citrate and zinc oxide and further zinc ion sources selected from zinc acetate, zinc gluconate, zinc glycinate, zinc sulfate, zinc phosphate and sodium zinc citrate. In certain embodiments, the weight ratio of zinc oxide to zinc citrate is from 0:5 to 5:1; from 0:5 to 4:1, or from 0:5 to 3:1. In some embodiments, the weight ratio of zinc oxide to zinc citrate is from 0.1:1 to 4:1, from 1:1 to 3:1, from 1.5:1 to 2.5:1, or 2:1. In some embodiments, the composition comprises from 0.1 to 5.0 weight % zinc oxide and from 0.1 to 2.0 weight % zinc citrate; from 0.1 to 2.0 weight % zinc oxide and from 0.20 to 1.0 weight % zinc citrate; from 0.5 to 1.5 weight % zinc oxide and from 0.25 to 0.75 weight % zinc citrate; from 0.75 to 1.25 weight % zinc oxide and from 0.4 to 0.6 weight % zinc citrate; or 1.0 weight % zinc oxide and 0.5 weight % zinc citrate.

In some embodiments, the composition is a dentifrice, a toothpaste, a gel, a tooth powder, a mouthwash, a mouthrinse, a lozenge, a tablet, a spray, a gum, or a film.

In a second aspect, the present invention also provides an oral care composition as described in any of the above embodiments, for use in reducing or inhibiting biofilm formation in an oral cavity.

In a third aspect, the present invention also provides a method of reducing or inhibiting biofilm formation in an oral cavity, the method comprising contacting the oral cavity with an oral care composition as described in any of the above embodiments.

In a fourth aspect, the present invention also provides for the use, in an oral care composition of the invention, of a combination of (a) a zinc ion source; and (b) at least two active ingredients to reduce or inhibit biofilm formation in an oral cavity. Furthermore, the composition may be a dentifrice, a toothpaste, a gel, a tooth powder, a mouthwash, a mouthrinse, a lozenge, a tablet, a spray, a gum, or a film.

In any embodiments of each of the above aspects, the oral care compositions may further comprise additional ingredients. These additional ingredients may include, but are not limited to, diluents, bicarbonate salts, surfactants, foam modulators, sweeteners, flavorants, pigments, antibacterial agents, anticaries agents, anticalculus or tartar control agents, polymers (such as xanthan gum, carboxymethylcellulose, carrageenan gum) and mixtures thereof.

In some embodiments, the oral care compositions of the present invention comprise at least one bicarbonate salt useful for example to impart a "clean feel" to teeth and gums due to effervescence and release of carbon dioxide. The one or more additional bicarbonate salts are optionally present in a total amount of 0.1 wt. % to 50 wt. %, for example 1 wt. % to 20 wt. %, by total weight of the composition.

The oral care compositions of the invention may also comprise at least one surfactant. Any orally acceptable surfactant, most of which are anionic, nonionic or amphoteric, can be used. One or more surfactants are optionally present in a total amount of 0.01 wt.% to 10 wt. %, for example, from 0.05 wt. % to 5 wt. %, or from 0.1 wt. % to 2 wt. % by total weight of the composition.

The oral care compositions of the invention may comprise at least one foam modulator, useful for example to increase amount, thickness or stability of foam generated by the composition upon agitation. One or more foam modulators are optionally present in a total amount of 0.1 wt. % to 10 wt. %, for example from 0.2 wt. % to 5 wt. %, or from 0.25 wt. % to 2 wt.%, by total weight of the composition.

The oral care compositions of the present invention may comprise at least one sweetener (such as, for example, sodium saccharin), useful for example to enhance taste of the composition. One or more sweeteners are optionally present in a total amount depending strongly on the particular sweetener(s) selected, but typically 0.005 wt.% to 5 wt.%, by total weight of the composition, optionally 0.005 wt.% to 0.2 wt.%, further optionally 0.05 wt.% to 0.1 wt.% by total weight of the composition.

The compositions of the present invention may also comprise at least one flavorant, useful for example to enhance taste of the composition. One or more flavorants are optionally present in a total amount of from 0.01 wt. % to 5 wt. %, for example, from 0.03 wt. % to 2.5 wt.%, optionally 0.05 wt.% to 1.5 wt.%, further optionally 0.1 wt.% to 0.3 wt.% by total weight of the composition.

The compositions of the invention may comprise at least one colorant. Colorants herein include pigments, dyes, lakes and agents imparting a particular luster or reflectivity such as pearling agents. Any orally acceptable colorant can be used. One or more colorants are optionally present in a total amount of from 0.001 wt.% to 20 wt.%, for example, from 0.01 wt.% to 10 wt. %, or from 0.1 wt. % to 5 wt.%, by total weight of the composition.

The oral care compositions may also comprise a fluoride ion source. Fluoride ion sources may be added to the compositions of the invention at a level of 0.001 wt. % to 10 wt. %, e.g., from 0.003 wt. % to 5 wt. %, 0.01 wt. % to 1 wt., or 0.05 wt. %. However, it is to be understood that the weights of fluoride salts to provide the appropriate level of fluoride ion will obviously vary based on the weight of the counter ion in the salt, and one of skill in the art may readily determine such amounts.

The compositions of the present invention may comprise a saliva stimulating agent useful, for example, in amelioration of dry mouth. One or more saliva stimulating agents are optionally present in saliva stimulating effective total amount.

The compositions of the present invention may include antisensitivity agents. Such agents may be added in effective amounts, e.g., from 1 wt. % to 20 wt. % by weight based on the total weight of the composition, depending on the agent chosen.

The composition of the invention may further comprise an antioxidant.

The compositions of the present invention may additionally optionally comprise a tartar control (anticalculus) agent.

### EXAMPLES

### Example 1

Compositions 1 to 9 were formulated, each of which contained 1 weight % zinc oxide, 0.5 weight % zinc citrate and two active ingredients selected from bisabolol, citral, carvacrol and thymol. For each combination of the aforementioned active ingredients, three dentifrices were formulated which differed only in the total concentration of the two active ingredients (the total concentration being 1000 ppm, 3000 ppm and 5000 ppm). The weight ratio of the two active ingredients for each particular combination was kept constant. The base dentifrice formulation was the same for each of compositions 1 to 9, with only the balance of water in the composition being adjusted to allow for the differences in concentration of the two active ingredients.

The dentifrice compositions 1 to 9 are shown in Table 1, below:

**Table 1**

| **Formula** | **Bisabolol (ppm)** | **Citral (ppm)** | **Carvacrol (ppm)** | **Thymol (ppm)** |
|---|---|---|---|---|
| **1** | 335.1 | 664.9 | | |
| **2** | 1005.3 | 1994.7 | | |
| **3** | 1675.5 | 3324.5 | | |
| **4** | 609.8 | | 390.2 | |
| **5** | 1829.4 | | 1170.6 | |
| **6** | 3049.0 | | 1951.0 | |
| **7** | | | 81.6 | 918.4 |
| **8** | | | 244.8 | 2755.2 |
| **9** | | | 408.0 | 4592.0 |

The Biofilm Growth Inhibition University of Manchester Model was used to determine the ability of the above dentifrices 1 to 9 to reduce oral biofilms. The ability of a Control A dentifrice (which had the same base formulation as compositions 1 to 9 and contained 1 weight % zinc oxide and 0.5 weight % zinc citrate, but contained no bisabolol, citral, carvacrol or thymol) and a placebo dentifrice (which had the same base formulation as compositions 1 to 9, but contained no zinc ion sources and no bisabolol, citral, carvacrol or thymol) to reduce oral biofilms was also tested.

The protocol for this model is as follows
(1) Dental plaque was collected from four healthy volunteers and pooled together as inoculum. The Optical Density of the inoculum was matched to 0.3 absorbance at 610nm.
(2) Sterile hydroxyapatite (HAP) disks were incubated under anaerobic conditions at 37°C for 24 hours with 1mL of sterile artificial saliva (with 0.01 weight% sucrose) and 1mL of pooled saliva in a 24 well microplate.
(3) For each test dentifrice (and for each control) a treatment solution of 1 part dentifrice: 2 parts sterile distilled water by weight was made up. Each freshly prepared treatment solution was added to three wells and allowed to contact the HAP disk therein for 10 minutes.
(4) The liquid phase of each well was then removed and was replaced by 2mL sterile artificial saliva.
(5) The disks were then maintained at 37°C under anaerobic conditions for 8 days.
(6) At intervals of 2, 4 and 8 days, the disks were collected aseptically and transferred to half-strength pre-reduced thioglycollate medium (4.5 mL per disk).
(7) 100µL of the dilution 10-4, 10-5 and 10-6 were plated in duplicates for each disk on Neomycin/Vancomycin (NV) Agar for Total Gram-negative Anaerobes.
(8) The plates were surface-spread using a sterile spreader and were incubated anaerobically at 37°C for 72 hours, after which time the number of colonies on each plate was counted.

The log10 CFU/ml (where CFU = colony forming units) for each composition was calculated. A lower Log10 CFU/ml indicates that the composition tested has greater efficacy in inhibiting biofilm growth.

The results of the tests are shown in Table 2, below:

**Table 2**

| **Formula** | **Avg log CFU/mL** |
|---|---|
| **1** | 6.43 |
| **2** | 6.36 |
| **3** | 6.09 |
| **4** | 6.50 |
| **5** | 6.38 |
| **6** | 6.17 |
| **7** | 6.44 |
| **8** | 6.42 |
| **9** | 6.27 |
| **Control A** | 6.70 |
| **Placebo** | 8.11 |

As can be seen from Table 2, all of the compositions 1 to 9 provided greater efficacy in reducing biofilm than the Control A formulation (which contained zinc oxide and zinc citrate, but no bisabolol, citral, carvacrol or thymol) and the Placebo.

### Example 2

Compositions 10 to 18 were formulated, each of which contained 1 weight % zinc oxide, 0.5 weight % zinc citrate and two active ingredients selected from bisabolol, carvacrol, oregano essential oil (an extract of oregano) and rosemary essential oil (an extract of rosemary). For each combination of the aforementioned active ingredients, three dentifrices were formulated which differed only in the total concentration of the two active ingredients (the total concentration being 1000 ppm, 3000 ppm and 5000 ppm). The weight ratio of the two active ingredients for each particular combination was kept constant. The base dentifrice formulation was the same for each of compositions 10 to 18, with only the balance of water in the composition being adjusted to allow for the differences in concentration of the two active ingredients.

The dentifrice compositions 10 to 18 are shown in Table 3, below:

**Table 3**

| **Formula** | **Bisabolol (ppm)** | **Carvacrol (ppm)** | **Oregano (ppm)** | **Rosemary (ppm)** |
|---|---|---|---|---|
| **10** | 185.2 | | 814.8 | |
| **11** | 555.6 | | 2444.4 | |
| **12** | 926.0 | | 4074.0 | |
| **13** | 20.4 | | | 979.6 |
| **14** | 61.2 | | | 2938.8 |
| **15** | 102.0 | | | 4898.0 |
| **16** | | 13.2 | | 986.8 |
| **17** | | 39.6 | | 2960.4 |
| **18** | | 66.0 | | 4934.0 |

The Biofilm Growth Inhibition University of Manchester Model was used to determine the ability of the above dentifrices 1 to 9 to reduce oral biofilms. The Control data for this test was not available.

The protocol for this model is as follows
(9) Dental plaque was collected from four healthy volunteers and pooled together as inoculum. The Optical Density of the inoculum was matched to 0.3 absorbance at 610nm.
(10) Sterile hydroxyapatite (HAP) disks were incubated under anaerobic conditions at 37°C for 24 hours with 1mL of sterile artificial saliva (with 0.01 weight% sucrose) and 1mL of pooled saliva in a 24 well microplate.
(11) For each test dentifrice (and for each control) a treatment solution of 1 part dentifrice: 2 parts sterile distilled water by weight was made up. Each freshly prepared treatment solution was added to three wells and allowed to contact the HAP disk therein for 10 minutes.
(12) The liquid phase of each well was then removed and was replaced by 2mL sterile artificial saliva.
(13) The disks were then maintained at 37°C under anaerobic conditions for 8 days.
(14) At intervals of 2, 4 and 8 days, the disks were collected aseptically and transferred to half-strength pre-reduced thioglycollate medium (4.5 mL per disk).
(15) 100µL of the dilution 10-4, 10-5 and 10-6 were plated in duplicates for each disk on Neomycin/Vancomycin (NV) Agar for Total Gram-negative Anaerobes.
(16) The plates were surface-spread using a sterile spreader and were incubated anaerobically at 37°C for 72 hours, after which time the number of colonies on each plate was counted.

The log10 CFU/ml (where CFU = colony forming units) for each composition was calculated. A lower Log10 CFU/ml indicates that the composition tested has greater efficacy in inhibiting biofilm growth.

The results of the tests are shown in Table 4, below:

**Table 4**

| **Formula** | **Avg log CFU/mL** |
|---|---|
| **10** | 4.98 |
| **11** | 4.72 |
| **12** | 4.54 |
| **13** | 4.94 |
| **14** | 4.65 |
| **15** | 4.46 |
| **16** | 4.86 |
| **17** | 4.52 |
| **18** | 4.38 |
| **Control** | Not Available |

As can be seen from Table 4, all of the compositions 1 to 9 provided excellent efficacy in reducing biofilm. The control data for this test was not available, but it is believed that the same improvement in efficacy would be achieved with compositions 10-18 as was achieved with compositions 1 to 9.

### Example 3

A commercially available zinc citrate/stannous chloride dentifrice that contained no bisabolol, citral, carvacrol, thymol, oregano essential oil or rosemary essential oil (Comparative Commercial Product) was tested for comparison.

**Table 5**

| | |
|---|---|
| **Comparative Commercial Product** | 6.49 |

Compositions 1-18 provided great efficacy in reducing biofilm over the commercially available zinc citrate/stannous chloride dentifrice which did not contain bisabolol, citral, carvacrol, thymol, oregano essential oil or rosemary essential oil.

## Claims

1. An oral care composition comprising:
(a) a zinc ion source, wherein the composition comprises from 0.1 to 2.0 weight % zinc oxide and from 0.20 to 1.0 weight % zinc citrate; and
(b) at least two active ingredients, wherein said active ingredients are selected from the following: bisabolol; citral; carvacrol; thymol; and an extract of oregano, wherein the active compound from said extract of oregano is selected from carvacrol, thymol, limonene, pinene, p-cymene, caryophyllene, and mixtures thereof; and an extract of rosemary, wherein the active compound from said extract of rosemary is selected from α-pinene, β-pinene, borneol, bornyl acetate, camphor, camphene, 1,8-cineole, limonene, and mixtures thereof.

2. The oral care composition of claim 1, wherein
(A) the total concentration of the zinc ion source is from 0.3 to 4.0 weight %, or from 0.5 to 2.0 weight %, based on the total weight of the composition; or
(B) the weight ratio of zinc oxide to zinc citrate is from 0:5 to 3:1; preferably wherein the weight ratio of zinc oxide to zinc citrate is from 1:1 to 3:1, optionally 2:1; or
(C) the composition comprises from 0.5 to 1.5 weight % zinc oxide and from 0.25 to 0.75 weight % zinc citrate.

3. The oral care composition of any preceding claim, wherein the total concentration of the at least two active ingredients is from 500 to 10,000 ppm, or from 1000 to 5000 ppm.

4. The oral care composition of any preceding claim, wherein the composition comprises
A) carvacrol; and/or
B) thymol; and/or
C) citral; and/or
D) bisabolol.

5. The oral care composition of claim 4, wherein the composition comprises bisabolol and citral; preferably, wherein the ratio of citral to bisabolol is from 1:1 to 3:1 by weight; optionally 2:1 by weight.

6. The oral care composition of any one of claims 1 to 3, wherein the composition comprises
a) bisabolol and carvacrol, preferably wherein the ratio of bisabolol to carvacrol is from 1:2 to 2:1 by weight; optionally 1.5:1 by weight; or
b) comprises bisabolol and thymol; or
c) comprises citral and carvacrol; or
d) comprises citral and thymol; or comprises carvacrol and thymol, preferably wherein the ratio of thymol to carvacrol is from 8:1 to 13:1 by weight; optionally 11.25:1 by weight.

7. The oral care composition of any one of claims 1 to 3, wherein the oral care composition comprises an active compound from an extract of oregano, and wherein the active compound from an extract of oregano is selected from carvacrol, thymol, limonene, pinene, p-cymene, caryophyllene, and mixtures thereof; preferably, wherein the active compound from the extract of oregano comprises 50 to 70 weight % carvacrol, 10 to 35 weight % thymol and 10 to 20 weight % p-cymene, based on the total weight of the extract of oregano.

8. The oral care composition of claim 7, wherein the composition further comprises bisabolol and wherein the ratio of the active compound from an extract of oregano to bisabolol is from 2:1 to 6:1 by weight; optionally 4.4:1 by weight.

9. The oral care composition of claim 7, wherein the composition further comprises citral.

10. The oral care composition of any one of claims 1 to 3, wherein the oral care composition comprises an active compound from an extract of rosemary, and wherein the active compound from the extract of rosemary is selected from α-pinene, β-pinene, borneol, bornyl acetate, camphor, camphene, 1,8-cineole, limonene, and mixtures thereof; preferably wherein the extract of rosemary comprises 25 to 40 weight % α-pinene, 5 to 20 weight % β-pinene, and 10 to 25 weight % camphor, based on the total weight of the extract of rosemary.

11. The oral care composition of claim 10, wherein the composition further comprises
i) bisabolol, preferably wherein the ratio of the active compound from an extract of rosemary to bisabolol is from 40:1 to 60:1 by weight; optionally 48:1 by weight; or
ii) citral; or
iii) carvacrol; preferably wherein the ratio of the active compound from an extract of rosemary to carvacrol is from 70:1 to 80:1 by weight; optionally 75:1 by weight; or
iv) thymol; or
v) an active compound from an extract of oregano; preferably wherein the active compound from an extract of oregano is selected from carvacrol, thymol, limonene, pinene, p-cymene, caryophyllene, and mixtures thereof; preferably wherein the composition comprises 50 to 70 weight % carvacrol, 10 to 35 weight % thymol and 10 to 20 weight % p-cymene, based on the weight of the extract of oregano.

12. The oral care composition of any one of the preceding claims, wherein the composition is a dentifrice, a toothpaste, a gel, a tooth powder, a mouthwash, a mouthrinse, a lozenge, a tablet, a spray, a gum, or a film.

13. The oral care composition of any preceding claim, for use in reducing or inhibiting biofilm formation in an oral cavity.

## Patentansprüche

1. Mundpflegezusammensetzung, umfassend:
(a) eine Zinkionenquelle, wobei die Zusammensetzung von 0,1 bis 2,0 Gewichts-% Zinkoxid und von 0,20 bis 1,0 Gewichts-% Zinkcitrat umfasst; und
(b) mindestens zwei aktive Inhaltsstoffe, wobei die aktiven Inhaltsstoffe aus den folgenden ausgewählt sind: Bisabolol; Citral; Carvacrol; Thymol; und einem Oreganoextrakt, wobei die aktive Verbindung von dem Oreganoextrakt ausgewählt ist aus Carvacrol, Thymol, Limonen, Pinen, p-Cymol, Caryophyllen und Mischungen davon; und einem Rosmarinextrakt, wobei die aktive Verbindung von dem Rosmarinextrakt ausgewählt ist aus α-Pinen, β-Pinen, Borneol, Bornylacetat, Campher, Camphen, 1,8-Cineol, Limonen und Mischungen davon.

2. Mundpflegezusammensetzung nach Anspruch 1, wobei
(A) die Gesamtkonzentration der Zinkionenquelle von 0,3 bis 4,0 Gewichts-% oder von 0,5 bis 2,0 Gewichts-%, bezogen auf das Gesamtgewicht der Zusammensetzung, beträgt; oder
(B) das Gewichtsverhältnis von Zinkoxid zu Zinkcitrat von 0:5 bis 3:1 beträgt; wobei das Gewichtsverhältnis von Zinkoxid zu Zinkcitrat vorzugsweise von 1:1 bis 3:1, wahlweise 2:1 beträgt; oder
(C) die Zusammensetzung von 0,5 bis 1,5 Gewichts-% Zinkoxid und von 0,25 bis 0,75 Gewichts-% Zinkcitrat umfasst.

3. Mundpflegezusammensetzung nach einem beliebigen vorhergehenden Anspruch, wobei die Gesamtkonzentration der mindestens zwei aktiven Inhaltsstoffe von 500 bis 10 000 ppm oder von 1 000 bis 5 000 ppm beträgt.

4. Mundpflegezusammensetzung nach einem beliebigen vorhergehenden Anspruch, wobei die Zusammensetzung umfasst
A) Carvacrol; und/oder
B) Thymol; und/oder
C) Citral; und/oder
D) Bisabolol.

5. Mundpflegezusammensetzung nach Anspruch 4, wobei die Zusammensetzung Bisabolol und Citral umfasst; vorzugsweise, wobei das Verhältnis von Citral zu Bisabolol von 1:1 bis 3:1, bezogen auf das Gewicht beträgt; wahlweise 2:1, bezogen auf das Gewicht.

6. Mundpflegezusammensetzung nach einem beliebigen der Ansprüche 1 bis 3, wobei die Zusammensetzung umfasst
a) Bisabolol und Carvacrol, wobei das Verhältnis von Bisabolol zu Carvacrol vorzugsweise von 1:2 bis 2:1, bezogen auf das Gewicht, wahlweise 1,5:1, bezogen auf das Gewicht, beträgt, oder
b) Bisabolol und Thymol umfasst; oder
c) Citral und Carvacrol umfasst; oder
d) Citral und Thymol umfasst; oder Carvacrol und Thymol umfasst, wobei das Verhältnis von Thymol zu Carvacrol vorzugsweise 8:1 bis 13:1, bezogen auf das Gewicht, wahlweise 11,25:1, bezogen auf das Gewicht, beträgt.

7. Mundpflegezusammensetzung nach einem beliebigen der Ansprüche 1 bis 3, wobei die Mundpflegezusammensetzung einen aktiven Inhaltsstoff von einem Oreganoextrakt umfasst, und wobei der aktive Inhaltsstoff von einem Oreganoextrakt ausgewählt ist aus Carvacrol, Thymol, Limonen, Pinen, p-Cymol, Caryophyllen und Mischungen davon; wobei der aktive Inhaltsstoff des Oreganoextrakts vorzugsweise 50 bis 70 Gewichts-% Carvacrol, 10 bis 35 Gewichts-% Thymol und 10 bis 20 Gewichts-% p-Cymol umfasst, bezogen auf das Gesamtgewicht des Oreganoextrakts.

8. Mundpflegezusammensetzung nach Anspruch 7, wobei die Zusammensetzung weiterhin Bisabolol umfasst und wobei das Verhältnis des aktiven Inhaltsstoffs von einem Oreganoextrakt zu Bisabolol von 2:1 bis 6:1, bezogen auf das Gewicht, beträgt; wahlweise 4,4:1, bezogen auf das Gewicht.

9. Mundpflegezusammensetzung nach Anspruch 7, wobei die Zusammensetzung weiterhin Citral umfasst.

10. Mundpflegezusammensetzung nach einem beliebigen der Ansprüche 1 bis 3, wobei die Mundpflegezusammensetzung einen aktiven Inhaltsstoff von einem Rosmarinextrakt umfasst, und wobei der aktive Inhaltsstoff von dem Rosmarinextrakt ausgewählt ist aus α-Pinen, β-Pinen, Borneol, Bornylacetat, Campher, Camphen, 1,8-Cineol, Limonen und Mischungen davon; wobei der Rosmarinextrakt vorzugsweise 25 bis 40 Gewichts-% α-Pinen, 5 bis 20 Gewichts-% β-Pinen und 10 bis 25 Gewichts-% Campher umfasst, bezogen auf das Gesamtgewicht des Rosmarinextrakts.

11. Mundpflegezusammensetzung nach Anspruch 10, wobei die Zusammensetzung weiterhin umfasst
i) Bisabolol, wobei das Verhältnis des aktiven Inhaltsstoffs von einem Rosmarinextrakt zu Bisabolol vorzugsweise von 40:1 bis 60:1, bezogen auf das Gewicht, wahlweise 48:1, bezogen auf das Gewicht, beträgt; oder
ii) Citral; oder
iii) Carvacrol, wobei vorzugsweise das Gewichtsverhältnis des aktiven Inhaltsstoffs eines Rosmarinextrakts zu Carvacrol von 70:1 bis 80:1, wahlweise 75:1, beträgt, oder
iv) Thymol; oder
v) einen aktiven Inhaltsstoff von einem Oreganoextrakt; wobei der aktive Inhaltsstoff von einem Oreganoextrakt vorzugsweise aus Carvacrol, Thymol, Limonen, Pinen, p-Cymol, Caryophyllen und Mischungen davon ausgewählt ist; wobei die Zusammensetzung vorzugsweise 50 bis 70 Gewichts-% Carvacrol, 10 bis 35 Gewichts-% Thymol und 10 bis 20 Gewichts-% p-Cymol umfasst, bezogen auf das Gewicht des Oreganoextrakts.

12. Mundpflegezusammensetzung nach einem beliebigen der vorhergehenden Ansprüche, wobei die Zusammensetzung ein Zahnputzmittel, eine Zahnpasta, ein Gel, ein Zahnpulver, ein Mundwasser, eine Mundspülung, eine Pastille, eine Tablette, ein Spray, ein Kaugummi oder ein Film ist.

13. Mundpflegezusammensetzung nach einem beliebigen der vorhergehenden Ansprüche zur Verwendung bei der Verringerung oder Hemmung der Biofilmbildung in einer Mundhöhle.

## Revendications

1. Composition de soin buccal comprenant :
(a) une source d'ions zinc, dans laquelle la composition comprend de 0,1 à 2,0 % en poids d'oxyde de zinc et de 0,20 à 1,0 % en poids de citrate de zinc ; et
(b) au moins deux principes actifs, lesdits principes actifs étant choisis parmi les suivants : le bisabolol ; le citral ; le carvacrol ; le thymol ; et un extrait d'origan, dans lequel le composé actif issu dudit extrait d'origan est choisi parmi le carvacrol, le thymol, le limonène, le pinène, le p-cymène, le caryophyllène et leurs mélanges ; et un extrait de romarin, dans lequel le composé actif issu dudit extrait de romarin est choisi parmi l'a-pinène, le β-pinène, le bornéol, l'acétate de bornyle, le camphre, le camphène, le 1,8-cinéole, le limonène et leurs mélanges.

2. Composition de soin buccal selon la revendication 1, dans laquelle
(A) la concentration totale de la source d'ions zinc est de 0,3 à 4,0 % en poids, ou de 0,5 à 2,0 % en poids, par rapport au poids total de la composition ; ou
(B) le rapport pondéral de l'oxyde de zinc au citrate de zinc est de 0:5 à 3:1 ; de préférence dans laquelle le rapport pondéral de l'oxyde de zinc au citrate de zinc est de 1:1 à 3:1, éventuellement de 2:1 ; ou
(C) la composition comprend de 0,5 à 1,5 % en poids d'oxyde de zinc et de 0,25 à 0,75 % en poids de citrate de zinc.

3. Composition de soin buccal selon l'une quelconque des revendications précédentes, dans laquelle la concentration totale des au moins deux principes actifs est de 500 à 10 000 ppm, ou de 1 000 à 5 000 ppm.

4. Composition de soin buccal selon l'une quelconque des revendications précédentes, dans laquelle la composition comprend
A) du carvacrol ; et/ou
B) du thymol ; et/ou
C) du citral ; et/ou
D) du bisabolol.

5. Composition de soin buccal selon la revendication 4, dans laquelle la composition comprend du bisabolol et du citral ; de préférence, dans laquelle le rapport du citral au bisabolol est de 1:1 à 3:1 en poids ; éventuellement de 2:1 en poids.

6. Composition de soin buccal selon l'une quelconque des revendications 1 à 3, dans laquelle la composition comprend
a) du bisabolol et du carvacrol, de préférence dans laquelle le rapport du bisabolol au carvacrol est de 1:2 à 2:1 en poids ; éventuellement de 1,5:1 en poids ; ou
b) comprend du bisabolol et du thymol ; ou
c) comprend du citral et du carvacrol ; ou
d) comprend du citral et du thymol ; ou comprend du carvacrol et du thymol, de préférence dans laquelle le rapport du thymol au carvacrol est de 8:1 à 13:1 en poids ; éventuellement de 11,25:1 en poids.

7. Composition de soin buccal selon l'une quelconque des revendications 1 à 3, dans laquelle la composition de soin buccal comprend un composé actif issu d'un extrait d'origan, et dans laquelle le composé actif issu d'un extrait d'origan est choisi parmi le carvacrol, le thymol, le limonène, le pinène, le p-cymène, le caryophyllène, et leurs mélanges ; de préférence, dans laquelle le composé actif issu de l'extrait d'origan comprend 50 à 70 % en poids de carvacrol, 10 à 35 % en poids de thymol et 10 à 20 % en poids de p-cymène, par rapport au poids total de l'extrait d'origan.

8. Composition de soin buccal selon la revendication 7, dans laquelle la composition comprend en outre du bisabolol et dans laquelle le rapport du composé actif issu d'un extrait d'origan au bisabolol est de 2:1 à 6:1 en poids ; éventuellement de 4,4:1 en poids.

9. Composition de soin buccal selon la revendication 7, dans laquelle la composition comprend en outre du citral.

10. Composition de soin buccal selon l'une quelconque des revendications 1 à 3, dans laquelle la composition de soin buccal comprend un composé actif issu d'un extrait de romarin, et dans laquelle le composé actif issu de l'extrait de romarin est choisi parmi l'a-pinène, le β-pinène , le bornéol, l'acétate de bornyle, le camphre, le camphène, le 1,8-cinéole, le limonène et leurs mélanges ; de préférence dans laquelle l'extrait de romarin comprend 25 à 40 % en poids d'a-pinène, 5 à 20 % en poids de β-pinène et 10 à 25 % en poids de camphre, par rapport au poids total de l'extrait de romarin.

11. Composition de soin buccal selon la revendication 10, dans laquelle la composition comprend en outre
i) du bisabolol, de préférence dans laquelle le rapport du composé actif issu d'un extrait de romarin au bisabolol est de 40:1 à 60:1 en poids ; éventuellement de 48:1 en poids ; ou
ii) du citral ; ou
iii) du carvacrol ; de préférence dans laquelle le rapport du composé actif issu d'un extrait de romarin au carvacrol est de 70:1 à 80:1 en poids ; éventuellement de 75:1 en poids ; ou
iv) du thymol ; ou
v) un composé actif issu d'un extrait d'origan ; de préférence dans laquelle le composé actif issu d'un extrait d'origan est choisi parmi le carvacrol, le thymol, le limonène, le pinène, le p-cymène, le caryophyllène, et leurs mélanges ; de préférence, dans laquelle la composition comprend 50 à 70 % en poids de carvacrol, 10 à 35 % en poids de thymol et 10 à 20 % en poids de p-cymène, par rapport au poids de l'extrait d'origan.

12. Composition de soin buccal selon l'une quelconque des revendications précédentes, dans laquelle la composition est un dentifrice, une pâte dentifrice, un gel, une poudre dentaire, un bain de bouche, un rince-bouche, une pastille, un comprimé, un spray, une gomme ou un film.

13. Composition de soin buccal selon l'une quelconque des revendications précédentes, pour une utilisation dans la réduction ou l'inhibition de la formation de biofilm dans une cavité buccale.
